# EUROPEAN PATENT SPECIFICATION

(11) **EP 2 100 892 B1**
(45) Date of publication and mention of the grant of the patent: **23.04.2014**
(21) Application number: 09163080.6
(22) Date of filing: 29.07.2005
(51) Int. Cl.: C07D 401/14

(54) **Preparation of pyridonecarboxylic acid antibacterials**
Herstellung von Pyridoncarboxylischer Säure-Antibakterien
Préparation de substances antibactériennes à base d'acide pyridonecarboxylique

(30) Priority: 30.07.2004 US 592893 P
(43) Date of publication of application: 16.09.2009
(62) Divisional of application: 05777596.7
(73) Proprietor: AbbVie Inc., North Chicago, IL 60064 (US)
(72) Inventor: Haight, Anthony, Wadsworth, IL 60083 (US); Barnes, David, Lake Villa, IL 60046 (US); Zhang, Geoff, Libertyville, IL 60048 (US)
(74) Representative: Modiano, Micaela Nadia

(56) References cited:
- EP-A- 0 339 406
- EP-A- 0 911 327
- US-A- 5 385 900
- US-A- 5 969 141
- MEALY N E ET AL: "ABT-492. Quinolone antibacterial." DRUGS OF THE FUTURE, vol. 27, no. 11, November 2002 (2002-11), pages 1033-1038, XP002356117 ISSN: 0377-8282
- ARAKI K ET AL: "Quinolone Antimicrobial Agents Substituted with Morpholines at the 7-Position. Syntheses and Structure-Activity Relationships" JOURNAL OF MEDICINAL CHEMISTRY, AMERICAN CHEMICAL SOCIETY, WASHINGTON, US, vol. 36, no. 10, 1 January 1993 (1993-01-01), pages 1356-1363, XP001037845 ISSN: 0022-2623
- BARNES, D.M. ET AL.: "Chlorination at the 8-position of a functionalized quinolone and the synthesis of quinolone antibiotic ABT-492", ORGANIC PROCESS RESEARCH AND DEVELOPEMENT, vol. 10, 21 June 2006 (2006-06-21), pages 803-807,

## Description

### FIELD OF THE INVENTION

This invention pertains to processes for preparing pyridonecarboxylic acid derivatives having antibacterial properties.

### BACKGROUND OF THE INVENTION

Many compounds having a pyridonecarboxylic acid moiety are known to be useful as antibacterials. For example, a series of novel quinolone antibacterials appears in PCT Application No. PCT/JP96/02710, published as WO97/11068 on March 27, 1997 and issued in United States as U.S. 5,998,436 on December 7, 1999, U.S. 6,133,284 on October 17, 2000, and U.S. 6,156,903 on December 5, 2000.

There is therefore an existing need for processes for making these compounds in large scale quantities.

### SUMMARY OF THE INVENTION

The present invention pertains to a process for making 1-(6-amino-3,5-difluoropyridin-2-yl)-8-chloro-6-fluoro- 7-(3-hydroxyazetidin-1-yl)-4-oxo-1,4-dihydroquinoline-3-carboxylic acid or a salt thereof, comprising:
(a) reacting ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide in ethyl acetate and in the presence of acid and isolating or not isolating the product;
   or
   reacting ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide in a mixture of methyl acetate and ethyl acetate and in the presence of sulfuric acid and isolating or not isolating the product; and
(b) reacting the product of step (a) and sodium hydroxide.

The process can further comprise isolating the product of step (b).

### DETAILED DESCRIPTION OF THE INVENTION

The present invention pertains to a process for making 1-(6-amino-3,5-difluoropyridin-2-yl)-8-chloro-6-fluoro- 7-(3-hydroxyazetidin-1-yl)-4-oxo-1,4-dihydroquinoline-3-carboxylic acid or a salt thereof, comprising:
(a) reacting ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide in ethyl acetate and in the presence of acid and isolating or not isolating the product;
   or
   reacting ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide in a mixture of methyl acetate and ethyl acetate and in the presence of sulfuric acid and isolating or not isolating the product; and
(b) reacting the product of step (a) and sodium hydroxide.

The process can further comprise isolating the product of step (b).

2,6-diamino-3,5-difluoropyridine, or a salt thereof can be made with a process comprising:
(a) reacting 2,3,5,6-tetrafluoropyridine, a compound having formula (I)

   H₂NCH(R¹)(R²) (I),

   or a salt thereof,
   wherein R¹ is phenyl or naphthyl, each of which is unsubstituted or substituted with one or two independently selected -O(CH₃) or -O(CH₂CH₃) substituents, and R² is hydrogen, -CH₃, -CH₂CH₃, or R¹ and a phosphate base at about 50°C to about 70°C for a first reaction time and at about 150°C to about 170°C for a second reaction time, wherein the total reaction time is about 16 hours to about 24 hours, to provide a compound having formula (II) 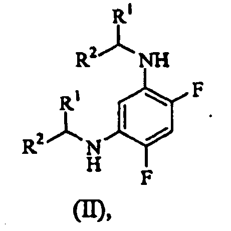 and isolating or not isolating the compound having formula (II);
(b) reacting, for about 45 minutes to about 2 hours, the compound having formula (II), hydrogen and a hydrogenolysis catalyst in water and a co-solvent, wherein the water is present in about 0.1 to about 6 molar equivalents per molar equivalent of the compound having formula (II).

2,6-diamino-3,5-difluoropyridine, or a salt thereof, can be made with a process comprising:
(a) reacting 2,3,5,6-tetrafluoropyridine, benzylamine and a phosphate base at about 50°C to about 70°C for a first reaction time and at about 150°C to about 170°C for a second reaction time, wherein the total reaction time is about 16 hours to about 24 hours, to provide N²,N⁶-dibenzyl-2,6-diamino-3,5-difluoropyridine;
(b) reacting, for about 45 minutes to about 2 hours, the N²,N⁶-dibenzyl-2,6-diamino-3,5-difluoropyridine, hydrogen and a hydrogenolysis catalyst in water and a co-solvent, wherein the water is present in about 0.1 to about 6 molar equivalents per molar equivalent of the N²,N⁶-dibenzyl-2,6-diamino-3,5-difluoropyridine.

2,6-diamino-3,5-difluoropyridine, or a salt thereof, can be made with a process comprising:
(a) reacting 2,3,5,6-tetrafluoropyridine, benzylamine and a plurality of potassium phosphate particles having a mean diameter of about 420 micrometers (40 mesh) to about 2000 micrometers (10 mesh) at about 50°C to about 70°C for a first reaction time and at about 150°C to about 170°C for a second reaction time, wherein the total reaction time is about 16 hours to about 24 hours, to provide N²,N⁶-dibenzyl-2,6-diamino-3,5-difluoropyridine;
(b) reacting for about 45 minutes to about 2 hours the N²,N⁶-dibenzyl-2,6-diamino-3,5-difluoropyridine, formic acid and 20% palladium on carbon in water and a co-solvent, wherein the water is present in about 0.1 to about 6 molar equivalents per molar equivalent of the N²,N⁶-dibenzyl-2,6-diamino-3,5-difluoropyridine.

2,6-diamino-3,5-difluoropyridine, or a salt thereof, can be made with a process comprising:
(a) reacting 2,3,5,6-tetrafluoropyridine, benzylamine and a plurality of potassium phosphate particles having a mean diameter of about 420 micrometers (40 mesh) to about 2000 micrometers (10 mesh) at about 50°C to about 70°C for about 15 minutes to about 5 hours and at about 150°C to about 170°C for about 10 hours to about 20 hours to provide N²,N⁶-dibenzyl-2,6-diamino-3,5-difluoropyridine.
(b) reacting, for about 45 minutes to about 2 hours, the N²,N⁶-dibenzyl-2,6-diamino-3,5-difluoropyridine, formic acid and 20% palladium on carbon in water and isopropyl acetate, wherein the water is present in about 1 to about 5 molar equivalents per molar equivalent of the N²,N⁶-dibenzyl-2,6-diamino-3,5-difluoropyridine.
   A compound having formula (V) 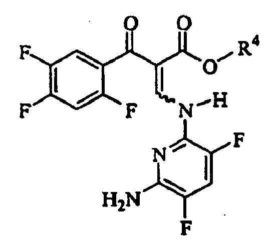 or a salt thereof, wherein R⁴ is alkyl, can be made with a process comprising:
(c) reacting, at about 100°C to about 140°C, a compound having formula (IV) 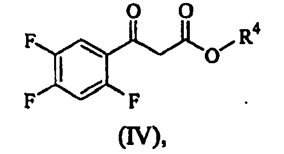 and a trialkylorthoformate for about 30 minutes to about 2 hours and reacting the product therefrom and a carboxylic anhydride for about 30 minutes to about 12 hours; and
(d) reacting the product of step (c) and 2,6-diamino-3,5-difluoropyridine to provide a product mixture comprising the compound having formula (V), mixing or not mixing the product mixture and water, and isolating or not isolating the compound having formula (V).

Ethyl (2E/Z)-3-((6-amino-3,5-difluoropyridin-2-yl)amino)-2-(2,4,5-trifluorobenzoyl)-2-propenoate, or a salt thereof, can be made with a process comprising:
(c) reacting, at about 100°C to about 140°C, ethyl 3-oxo-3-(2,4,5-trifluorophenyl)propanoate and a trialkylorthoformate for about 30 minutes to about 2 hours and reacting the product therefrom and a carboxylic anhydride for about 30 minutes to about 12 hours; and
(d) reacting the product of step (c) and 2,6-diamino-3,5-difluoropyridine to provide a product mixture comprising ethyl (2E/Z)-3-((6-amino-3,5-difluoropyridin-2-yl)amino)-2-(2,4,5-trifluorobenzoyl)-2-propenoate, mixing or not mixing the product mixture and water, and isolating or not isolating the ethyl (2E/Z)-3-((6-amino-3,5-difluoropyridin-2-yl)amino)-2-(2,4,5-trifluorobenzoyl)-2-propenoate.

Ethyl (2E/Z)-3-((6-amino-3,5-difluoropyridin-2-yl)amino)-2-(2,4,5-trifluorobenzoyl)-2-propenoate, or a salt thereof, can be made with a process comprising:
(c) reacting, at about 100°C to about 140°C, ethyl 3-oxo-3-(2,4,5-trifluorophenyl)propanoate and triethylorthoformate for about 30 minutes to about 2 hours and reacting the product therefrom and acetic anhydride for about 30 minutes to about 12 hours; and
(d) reacting the product of step (c) and 2,6-diamino-3,5-difluoropyridine to provide a product mixture comprising the ethyl (2E/Z)-3-((6-amino-3,5-difluoropyridin-2-yl)-amino)-2-(2,4,5-trifluorobenzoyl)-2-propenoate, mixing or not mixing the product mixture and water, and isolating or not isolating the ethyl (2E/Z)-3-((6-amino-3,5-difluoropyridin-2-yl)amino)-2-(2,4,5-trifluorobenzoyl)-2-propenoate.

Ethyl (2E/Z)-3-((6-amino-3,5-difluoropyridin-2-yl)amino)-2-(2,4,5-trifluorobenzoyl)-2-propenoate, or a salt thereof, can be made with a process comprising:
(c) reacting, at about 110°C to about 115°C, ethyl 3-oxo-3-(2,4,5-trifluorophenyl)propanoate and triethylorthoformate for about 30 minutes to about 2 hours and reacting the product therefrom and acetic anhydride for about 30 minutes to about 12 hours; and
(d) reacting the product of step (c) and 2,6-diamino-3,5-difluoropyridine to provide a product mixture comprising the ethyl (2E/Z)-3-((6-amino-3,5-difluoropyridin-2-yl)amino)-2-(2,4,5-trifluorobenzoyl)-2-propenoate, mixing or not mixing the product mixture and water, and isolating or not isolating the ethyl (2E/Z)-3-((6-amino-3,5-difluoropyridin-2-yl)amino)-2-(2,4,5-trifluorobenzoyl)-2-propenoate.

Ethyl (2E/Z)-3-((6-amino-3,5-difluoropyridin-2-yl)amino)-2-(2,4,5-trifluorobenzoyl)-2-propenoate, or a salt thereof, can be made with a process comprising:
(c) reacting, at about 110°C to about 115°C, ethyl 3-oxo-3-(2,4,5-trifluorophenyl)propanoate and triethylorthoformate for about 30 minutes to about 2 hours and reacting the product therefrom and acetic anhydride for about 30 minutes to about 12 hours; and
(d) reacting the product of step (c) and 2,6-diamino-3,5-difluoropyridine to provide a product mixture comprising the ethyl (2E/Z)-3-((6-amino-3,5-difluoropyridin-2-yl)amino)-2-(2,4,5-trifluorobenzoyl)-2-propenoate, mixing the product mixture and water, and isolating the ethyl (2E/Z)-3-((6-amino-3,5-difluoropyridin-2-yl)amino)-2-(2,4,5-trifluorobenzoyl)-2-propenoate.
   Substantially pure 3-azetidinol hydrochloride can be made with a process comprising:
(e) reacting (±)-2-(chloromethyl)oxirane, sodium bicarbonate and the compound having formula (I) to provide a compound having formula (VI), 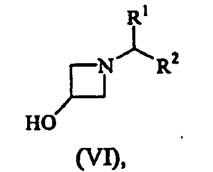 reacting the compound having formula (VI) and either hydrogen chloride at about -10°C to about 0°C or (2E/Z)-2-butenedioic (maleic) acid at about 35°C to about 45°C, and isolating either the hydrochloride salt or the maleate acid salt, respectively, of the compound having formula (VI);
(f) reacting or not reacting the product of step (e) and a base; and
(g) reacting the product of step (f), hydrogen and a hydrogenolysis catalyst in a reaction medium consisting essentially of water, an alcohol, and an organic acid, at about 40 psi to about 60 psi and at about 50°C to about 70°C, for about two hours to about four hours, and isolating the product, with the proviso that when the product of step (e) is the maleate salt, step (f) is conducted.

Substantially pure 3-azetidinol hydrochloride can be made with a process comprising:
(e) reacting (±)-2-(chloromethyl)oxirane, sodium bicarbonate and benzylamine to provide 1-benzyl-3-azetidinol, reacting the 1-benzyl-3-azetidinol and (2E/Z)-2-butenedioic acid at about 35°C to about 45°C, and isolating 1-benzyl-3-azetidinol (2E/Z)-2-butenedioate;
(f) reacting the product of step (e) and potassium carbonate; and
(g) reacting the product of step (f), hydrogen and Pd(OH)₂ on carbon in a reaction medium comprising water, an alcohol and an organic acid at about 40 psi to about 60 psi and about 50°C to about 70°C for about two hours to about four hours, reducing the water in the reaction medium to less than about 4 mg per mL and treating the reaction medium first with hydrogen chloride gas between about 0°C and about 20°C and then with toluene at about 40°C.

Substantially pure 3-azetidinol hydrochloride can be made with a process comprising:
(e) reacting (±)-2-(chloromethyl)oxirane, sodium bicarbonate and benzylamine in acetonitrile and water to provide 1-benzyl-3-azetidinol, reacting the 1-benzyl-3-azetidinol and (2E/Z)-2-butenedioic acid at about 35°C to about 45°C, and isolating 1-benzyl-3-azetidinol (2E/Z)-2-butenedioate;
(f) reacting the product of step (e) and potassium carbonate; and
(g) reacting the product of step (f), hydrogen and Pd(OH)₂ on carbon in a reaction medium comprising water, isopropanol and acetic acid at about 40 psi to about 60 psi and about 50°C to about 70°C for about 2 to about 4 hours, reducing the water in the reaction medium to less than about 4 mg per mL, and treating the reaction medium first with hydrogen chloride gas between about 0°C and about 20°C and then with toluene at about 40°C.

Substantially pure 3-azetidinol hydrochloride can be made with a process comprising:
(e) reacting (±)-2-(chloromethyl)oxirane, sodium bicarbonate and benzylamine at about 0°C to about 80°C in acetonitrile and water to provide 1-benzyl-3-azetidinol, reacting the 1-benzyl-3-azetidinol and (2E/Z)-2-butenedioic acid at about 40°C, and isolating 1-benzyl-3-azetidinol (2E/Z)-2-butenedioate;
(f) reacting the 1-benzyl-3-azetidinol (2E/Z)-2-butenedioate and potassium carbonate; and
(g) reacting the product of step (f), hydrogen and Pd(OH)₂ on carbon in a reaction medium comprising water, isopropanol and acetic acid at about 40 psi and about 65°C for about two hours, reducing the water in the reaction medium to less than about 4 mg per mL and treating the reaction medium first with hydrogen chloride gas between about 0°C and about 20°C and then with toluene at about 40°C.
   A compound having formula (VII) 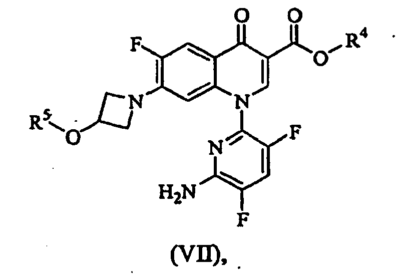 or a salt thereof,
   wherein R⁵ is hydrogen or C(O)R⁶, wherein R⁶ is alkyl, phenyl or naphthyl, each of which is unsubstituted or substituted with one or two or three of independently selected OCH₃, OCH₂CH₃, F, Cl or Br, can be made with a process comprising:
(h) reacting the compound having formula (V) or a salt thereof, a non-protic acid and a bicyclic amine base;
(i) reacting the product of step (h), 3-azetidinol hydrochloride and the bicyclic amine base and isolating or not isolating the product; and
(j) reacting or not reacting the product of step (i) and a OH protecting group precursor and isolating or not isolating the product.
   A compound having formula (VII), or a salt thereof, can be used for preparing 1-(6-amino-3,5-difluoropyridin-2-yl)-8-chloro-6-fluoro-7-(3 (isobutyryloxy)azetidin-1-yl)-4-oxo-1,4-dihydro-3-quinolinecarboxylic acid, 1-(6-amino-3,5-difluoropyridin-2-yl)-8-chloro-6-fluoro-7-(3-hydroxy-1-azetidinyl)-4-oxo-1,4-dihydro-3-quinolinecarboxylic acid, or a salt, ester or salt of an ester thereof.
   A compound having formula (VIIa) 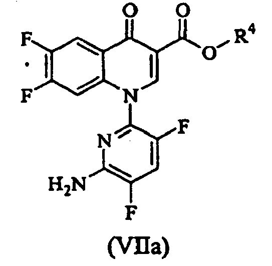 can be prepared as set forth in the preceeding embodiment.

Ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate, or a salt thereof, can be made with a process comprising:
(h) reacting ethyl (2E/Z)-3-((6-amino-3,5-difluoropyridin-2-yl)amino)-2-(2,4,5-trifluorobenzoyl)-2-propenoate, or a salt thereof, a non-protic acid and a bicyclic amine base and not isolating or isolating the product;
(i) reacting the product of step (h), 3-azetidinol hydrochloride and a bicyclic amine base and not isolating the product; and
(j) reacting the product of step (i) and isobutyric anhydride and isolating or not isolating the product.

Ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate, or a salt thereof, can be prepared as set forth in the preceeding .

Ethyl 1-(6-amino-3,5-difluoropyridin-2-yl)-6-fluora-7-(3-(isobutyryloxy)azetidin-1-yl)-4-oxo-1,4-dihydroquinoline-3-carboxylate, or a salt thereof, can be used for preparing 1-(6-amino-3,5-difluoropyridin-2-yl)-8-chloro-6-fluoro-7-(3-hydroxyazetidin-1-yl)-4-oxo-1,4-dihydro-3-quinolinecarboxylic acid, or a salt, ester or salt of an ester thereof.

Ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate, or a salt thereof, can be made with a process comprising:
(h) reacting ethyl (2E/Z)-3-((6-amino-3,5-difluoropyridin-2-yl)amino)-2-(2,4,5-trifluorobenzoyl)-2-propenoate, or a salt thereof, lithium chloride and DBU and not isolating the product;
(i) reacting the product of step (h), 3-azetidinol hydrochloride and DBU and not isolating the product; and
(j) reacting the product of step (i) and isobutyric anhydride and isolating or not isolating the product.
   Ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate, or a salt thereof, prepared as set forth in the preceeding embodiment.
   Ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate, or a salt thereof, can be used for preparing 1-(6-amino-3,5-difluoropyridin-2-yl)-8-chloro-6-fluoro-7-(3-hydroxyazetidin-1-yl)-4-oxo-1,4-dihydro-3-quinolinecarboxylic acid, or a salt, ester or salt of an ester thereof.
   1-(6-amino-3,5-difluoropyridin-2-yl)-8-chloro-6-fluoro-7-(3-hydroxyazetidin-1-yl)-4-oxo-1,4-dihydro-3-quinolinecarboxylic acid, or a salt thereof, can be made with a process comprising:
(k) reacting the compound having formula (VII), or a salt thereof, and a chlorinating agent and isolating or not isolating a compound having formula (VIII) 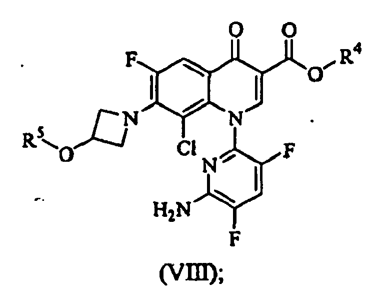
(l) reacting the product of step (k) and a hydroxide base; and
(m) isolating the product of step (1).

A compound having formula (VIII), or a salt thereof can be used for the preparation of 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof.

1-(6-amino-3,5-difluoropyndin-2-yl)-8-chloro-6-fluoro-7-(3-hydroxyazetidin-1-yl)-4-oxo-1,4-dihydroquinoline-3-carboxylic acid, or a salt thereof can be made with a process comprising:
(k) reacting ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide and isolating or not isolating the product;
(I) reacting the product of step (k) and sodium hydroxide; and
(m) isolating the product of step (I).

Variable moieties are represented by identifiers (capital letters with numerical and/or alphabetical superscripts) and may be specifically embodied.

The term "alkyl," as used herein, means C₁-alkyl, C₂-alkyl, C₃-alkyl, C₄-alkyl, C₅-alkyl, and C₆-alkyl.

The term "C₁-alkyl," as used herein, means methyl.

The term "C₂-alkyl," as used herein, means ethyl.

The term "C₃-alkyl," as used herein, means grap-1-yl and prop-2-yl (isopropyl).

The term "C₄-alkyl," as used herein, means but-1-yl, but-2-yl, 2-methylprop-1-yl, and 2-methylprop-2-yl (tert-butyl).

The term "C₅-alkyl," as used herein, means 2,2-dimethylprop-1-yl (neo-pentyl), 2-methylbut-1-yl, 2-methylbut-2-yl, 3-methylbut-1-yl, 3-methylbut-2-yl, pent-1-yl, pent-2-yl, and pent-3-yl.

The term "C₆-alkyl," as used herein, means 2,2-dimethylbut-1-yl, 2,3-dimethylbut-1-yl, 2,3-dimethylbut-2-yl, 3,3-dimethylbut-1-yl, 3,3-dimethylbut-2-yl, 2-ethylbut-1-yl, hex-1-yl, hex-2-yl, hex-3-yl, 2-methylpent-1-yl, 2-methylpent-2-yl, 2-methylpent-3-yl, 3-methylpent-1-yl, 3-methylpent-2-yl, 3-methylpent-3-yl, 4-methylpent-1-yl, and 4-methylpent-2-yl.

The term "alcohol," as used herein, means methanol, ethanol, isopropanol, tert-butanol, and the like or a mixture thereof.

The term "alkanoyl halide," as used herein, means a compound having formula . R⁶C(O)Cl, wherein R⁶ is alkyl which is unsubstituted or substituted with one or two or three of independently selected OCH₃, OCH₂CH₃, F, Cl or Br.

The term "aryloyl halide," as used herein, means a compound having formula R⁶C(O)Cl, wherein R⁶ is phenyl or naphthyl, each of which is unsubstituted or substituted with one or two or three of independently selected OCH₃, OCH₂CH₃, F, Cl or Br.

The effect of the solubility of compounds having formula (VII), wherein R⁴ is ethyl and R⁶ is variable, in ethyl acetate, is shown in TABLE 1.

| R⁶ | solubility (mg/mL) |
|---|---|
| CH₃ | 1.7 |
| CH₂CH₃ | 0.2 |
| CH(CH₃)₂ | 6.4 |
| phenyl | 0.2 |

The term "amine base," as used herein, means triethylamine, N-methylmorpholine, and diisopropylethylamine.

The term "base," as used herein, means Na₂CO₃, NaHCO₃, K₂CO₃, KHCO₃, triethylamine, diisopropylethylamine and the like, or a mixture thereof.

The term "bicyclic amine base," as used herein, means 1,5-diazabicyclo-[4,3,0]non-5-ene (DBN) and 1,8-diazabicyclo[5.4.0]undec-7-ene (DBN).

The term "carboxylic anhydride," as used herein, means acetic anhydride, butyric anhydride, isobutyric anhydride and the like.

The term "chlorinating agent," as used herein, means N-chlorosuccinimide, with or without water and with an acid such as sulfuric acid, phosphoric acid, trifluoroacetic acid, perchloric acid and the like. The yields of chlorinations of compounds having formula (VII), wherein R⁴ is CH₂CH₃ and R⁵ is C(O)R⁶, wherein R⁶ is CH(CH₃)₂, with N-chlorosuccinimide in ethyl acetate is shown in TABLE 2.

| acid/additive | amount (equivalents) | yield (%) |
|---|---|---|
| TFA/water | 0.1-0.2 | 83 |
| HClO₄ | 0.1-0.2 | 95 |
| H₃PO₄ | 0.1 | 90 |
| H₂SO₄ | 0.065 | 90 |

The term "hydrogenolysis catalyst," as used herein, means water-wet or not water-wet 5% palladium hydroxide, water-wet or not water-wet 10% palladium hydroxide, water-wet or not water-wet 5% palladium hydroxide on carbon, water-wet or not water-wet 10% palladium hydroxide on carbon, and the like or mixtures thereof.

The term "hydroxide base," as used herein, means the hydroxide base of sodium, potassium, lithium, barium and the like or mixtures thereof.

The term "non-protic acid," as used herein, means lithium chloride, magnesium chloride, zinc chloride and the like, or mixtures thereof.

The term "OH protecting group precursor," as used herein, means a carboxylic anhydride, an alkanoyl halide, an aryloyl chloride and the like.

The term "organic acid," as used herein, means formic acid, acetic acid, propionic acid and the like, or mixtures thereof.

The term "phosphate base," as used herein, means K₃PO₄, K₂HPO₄, KH₂PO₄, Na₃PO₄, Na₂HPO₄, NaH₂PO₄, and the like or mixtures thereof.

The term "substantially pure 3-azetidinol hydrochloride," as used herein, means 3-azetidinol hydrochloride having a low enough solvent content to be in powder form.

The term "trialkylorthoformate" means trimethylorthoformate, triethylorthoformate, triisopropylorthoformate and the like, or mixtures thereof.

Compounds mentioned herein can have one or more than one asymmetrically substituted carbon atoms in the R or S configuration. Compounds having asymmetrically substituted carbon atoms enriched with one configuration over the other are assigned the configuration which is present in the higher amount, preferably 85% to 95% enrichment, more preferably 95% to 99% enrichment, and still more preferably greater than 99% enrichment. Accordingly, compounds of this invention can exist as enantiomers, mixtures of enantiomers, diastereomers having relative stereochemistry, diastereomers having absolute stereochemistry, diastereomers having at least one asymmetrically substituted carbon atom which is enriched in one configuration and at least one asymmetrically substituted carbon atom which is not enriched, and mixtures of the preceeding.

Compounds mentioned herein can also have one or more than one carbon-carbon double bond or carbon-nitrogen double bond. Accordingly, compounds of this invention can exist as geometric isomers of either Z or E configuration or as mixtures of geometric isomers.

The terms "R", "S", "Z", and "E" are as defined by the IUPAC 1974 Recommendations for Section E, Fundamental Stereochemistry, Pure Appl. Chem. (1976) 45, 13-10.

Compounds mentioned herein may exist as acid addition salts or base addition salts and may be prepared during their isolation or following their purification. Acid addition salts of compounds are prepared by reaction with acid. For example, the acetate, adipate, alginate, bicarbonate, citrate, aspartate, benzoate, benzenesulfonate, bisulfate, butyrate, camphorate, camphorsufonate, citrate, digluconate, formate, fumarate, glycerophosphate, glutamate, hemisulfate, heptanoate, hexanoate, hydrochloride, hydrobromide, hydroiodide, lactobionate, lactate, maleate, mesitylenesulfonate, methanesulfonate, naphthylenesulfonate, nicotinate, oxalate, pamoate, pectinate, persulfate, phosphate, picrate, propionate, succinate, tartrate, thiocyanate, trichloroacetate, trifluoroacetate, para-toluenesulfonate, and undecanoate salts of compounds of this invention are meant to be embraced thereby. Base addition salts of compounds of this invention may be prepared by reaction with a base such as the hydroxide, carbonate, bicarbonate, phosphate, hydrogen phosphate, or dihydrogen phosphate of cations such as calcium, iron, lithium, potassium, sodium or magnesium.

The following examples are meant to further embody the processes of this invention.

### Reference EXAMPLE 1

A solution of benzylamine (73Kg) in water (650.4Kg) at 0°C was treated with epichlorohydrin (61 Kg), stirred for approximately 2 hours until solid formed, stored at 10°C for 16 hours, and filtered. The filtrant was mixed with sodium bicarbonate (104Kg) in acetonitrile (1110Kg), and the mixture was azeotropically distilled with acetonitrile addition to maintain a volume of 900L, diluted with acetonitrile (400L), stirred at 75°C for 10-16 hours, cooled to ambient temperature, filtered through diatomaceous earth (Celite®), concentrated to 300L, added over 1 hour to a solution of maleic acid (52.8Kg) in acetonitrile (310Kg) at 40°C, cooled to 0°C, and filtered. The filtrant was washed with isopropyl acetate and dried to provide 113.6Kg of product. Mp 127-129°C; ¹H NMR (CD₃OD) δ 7.54-7.51 (m, 5H), 6.33 (s, 2H), 4.98 (brs, 4H, exchangeable), 4.72 (quintet, J=6 Hz, 1H), 4.45 (s, 2H), 4.39 (m, 2H), 4.01 (m, 2H), 3.38 (CHD₂OD).

### Reference EXAMPLE 2

A suspension of EXAMPLE 1 (111.6Kg) in ethyl acetate (605Kg) was treated with 25% aqueous potassium carbonate (560Kg) until the suspension homogenized. The orgainc layer was isolated and concentrated with an isopropanol azeotrope. The concentrate was mixed with acetic acid (25.8Kg), added to half-wet 5% palladium hydroxide on carbon (13.1Kg), stirred at 55-65°C under hydrogen at 40 psi for 2-8 hours, cooled to ambient temperature, filtered, washed with isopropanol, concentrated with an isopropanol azeotrope to 110L, cooled to 5-10°C, treated with HCl gas (14Kg), stirred at ambient temperature for 1 hour and at 40°C for 30 minutes, treated with toluene (210Kg) over 1 hour, stirred for 30 minutes, cooled to ambient temperature, and filtered. The filtrant was washed with toluene and dried under vacuum at 50°C to provide 36.7Kg of product. ¹H NMR (CD₃OD) δ 4.57 (m, 1H), 4.08 (m, 2H), 3.80 (m, 2H), 3.38 (CHD₂OD).

### Reference EXAMPLE 3

A suspension of benzylamine (106Kg) and milled (20 mesh) potassium phosphate (45Kg) in N-methylpyrrolidinone (100Kg) at 50°C was treated with 2,3,5,6-tetrafluoropyridine (30Kg), stirred for 30 minutes at 50-70°C and at 165°C for 12-18 hours, cooled to 10°C, treated sequentially with water (240L) and 50% (v/v) isopropyl acetate/heptane (240L), isopropyl acetate (37.9 Kg), and water (88.5 Kg), each at 10°C. The bottom and middle layers were separated and washed with 2M HCl (120L) and water (120L), each precooled to 10°C, and concentrated. The concentrate was treated with isopropyl acetate (275Kg) and stored in an opaque container under nitrogen. ¹H NMR (CDCl₃, 300 MHz) δ 7.29-7.24 (m, 10H), 6.97 (t, 1H), 4.57 (s, 4H).

### Reference EXAMPLE 4

A mixture of half-wet 20% palladium hydroxide on carbon (68.1Kg) was treated sequentially with 39.1% (w/w) EXAMPLE 3 in isopropyl acetate (410Kg total, 160Kg EXAMPLE 3), isopropyl acetate (550Kg) and 88% formic acid (57Kg). The mixture was stirred at 50°C for 2 hours and filtered under nitrogen through diatomaceous earth (Celite®) with isopropyl acetate (200Kg) rinsing. The filtrate was washed twice with 6% citric acid solution having its pH adjusted to 4 with potassium hydroxide (water (200Kg)/citric acid (12Kg)/potassium hydroxide (2Kg)), 1M sodium bicarbonate solution (150Kg) and water (150Kg) and concentrated. The concentrate was treated with heptane(1005Kg) over 90 minutes, and the solution was cooled to 0°C and filtered. The filtrant was washed with heptane (220Kg) and dried under vacuum at 40°C to provide 61.5Kg of product, which was stored in an opaque container under nitrogen. ¹H NMR (CDCl₃, 300 MHz) δ 7.03 (t, 1H), 4.5-4.0 (brs, 4H).

### Reference EXAMPLE 5

A solution of 2,4,5-trifluorobenzoic acid (139.5Kg) in DMF (8.4Kg) and toluene (613Kg) was treated with thionyl chloride (139.4Kg), stirred at 60°C for 3.5 hours, cooled to 25°C, concentrated to 20% of its original volume, treated with toluene (600Kg), distilled and stored at ambient temperature.

### Reference EXAMPLE 6

A suspension of potassium ethyl malonate (50.8Kg) and magnesium chloride (34.5Kg) in toluene (130Kg) below 0°C was treated with THF (265L), cooled to 0°C, treated with triethylamine (75Kg), warmed to 50°C, stirred for 1-5 hours, cooled to 0°C, treated with 22% (w/w) of EXAMPLE 5 in toluene (163Kg), warmed to ambient temperature, stirred for 2 hours, added to 2M HCl (407Kg), stirred for 30 minutes, separated from the water layer and washed with water. This procedure was repeated, and the organic layers were combined, concentrated with an ethanol (150L) azeotrope, treated with water (30% by weight of the organic layer), stirred for 3 hours at 0°C, and filtered. The andfiltrant was washed with 3:1 ethanol/water and dried under vacuum at 35-45°C to provide 86Kg of product. ¹H NMR (CDCl₃) (keto) δ 7.75 (ddd, J=10.8, 10.8, 6.0Hz, 1H), 7.02 (ddd, 1H), 4.27 (q, J=7.2Hz, 2H), 3.95 (d, 4.2Hz, 2H), 1.35 (t, J=7.3Hz, 3H); (enol) δ 12.72 (s, 1H), 7.85 (ddd, J=10.5, 9.6, 6.6Hz, 1H), 6.96 (ddd, J=10.5, 10.5, 6.6Hz, 1H), 5.84 (s, 1H), 4.23 (q, J=7.2Hz, 2H), 1.27 (t, J=7.4Hz, 3H).

### Reference EXAMPLE 7A

A solution of EXAMPLE 6 (83.2Kg) in triethyl orthoformate (80.1Kg) at reflux was stirred for 0.5-1 hour, treated with acetic anhydride (103.5Kg), stirred for 12 hours and cooled to ambient temperature to provide a solution that was used immediately.

### Reference EXAMPLE 7B

The solution of EXAMPLE 7A was treated with N-methylpyrrolidinone (210Kg), acetonitrile (161Kg) and water (3Kg), added to a suspension of EXAMPLE 4 (57.4Kg) in 1:1 N-methylpyrrolidinone (210Kg) and acetonitrile (161Kg), stirred for 2 hours, added to water (662Kg) and filtered. The filtrant was washed with (2:1) acetonitrile/water and water and dried under vacuum at 60°C to provide 119.5Kg of product. Mp 157-160°C; ¹H NMR (CDCl₃, 300 MHz) (E) δ 1.15 (t, 3H), 4.16 (q, 2H), 4.64 (br s, 2H), 6.90 (m, 1H), 7.22 (t, 1H), 7.32 (m, 1H), 9.03 (d, 1H), 12.44 (bd, 1H); (Z) δ 1.03 (t, 3H), 4.11 (q, 2H), 4.60 (br s, 2H), 6.90 (m, 1H), 7.20 (t, 1H), 7.48 (m, 1H), 8.90 (d, 1H), 11.17 (bd, 1H).

### Reference EXAMPLE 8A

A mixture of EXAMPLE 7 (115Kg) and lithium chloride (24.3Kg) in N-methylpyrrolidinone (769Kg) below 35°C was treated with DBU (946.1Kg) and stirred for 2 hours to provide a solution of EXAMPLE 8A that was used immediately.

### Reference EXAMPLE 8B

The solution of EXAMPLE 8A below 40°C was treated with EXAMPLE 2 (33.9Kg) and DBU (109Kg) and stirred for 2-5 hours to provide a solution of EXAMPLE 8B that was used immediately.

### Reference EXAMPLE 8C

The solution of EXAMPLE 8B was treated with isobutyric anhydride (99.7Kg), stirred at 35°C for 1-2 hours, cooled to 20-30°C, treated with ethyl acetate (104Kg) and 10% aqueous citric acid (570Kg) and filtered. The filtrant was washed with water and dried under vacuum at 50°C to provide 136Kg of product. ¹H NMR (DMSO4-d₆, 400 MHz) δ 8.49 (s, 1H), 8.00 (dd, J=9.0, 9.3 Hz, 1H), 7.75 (d, J=12.8 Hz, 1H), 6.79 (br s, 2H), 5.95 (dd, J=1.5, 7.6 Hz, 1H), 5.21 (m, 1H), 4.36 (t, J=7.4 Hz, 2H), 4.02 (q, J=7.0 Hz, 2H), 3.95 (dd, J=3.7, 9.2 Hz, 2H), 2.58 (hept, J=7.0 Hz, 1H), 1.26 (t, J=7.0 Hz, 3H), 1.11 (d, J=7.0 Hz, 6H).

### Reference EXAMPLE 9A

A suspension of EXAMPLE 8 (99.8Kg) in dichloromethane (813Kg) at 0-5°C was treated with 1,3-dichloro-5,5-dimethylhydantoin (39.5Kg) in dichloromethane (540Kg) over 2 hours then with 10% aqueous sodium bisulfite (550Kg), separated from the water layer, washed with 5% sodium bicarbonate and water and concentrated. The concentrate was dissolved in methyl tert-butyl ether, crystallized at 5°C and dried at 65°C to provide 109 Kg of product as the methyl tert-butyl ether solvate. ¹H NMR (CDCl₃) methyl tert-butyl ether solvate: δ 8.35 (s, 1H), 7.95 (d, J=14.7Hz, 1H), 7.24 (t, J=8.9Hz, 1H), 5.19-5.11 (m, 1H), 4.82-4.72 (m, 2H), 4.39-4.27 (m, 2H), 4.35 (q, J=7.5Hz, 2H), 3.19 (s, 3H), 2.57 (sept, J=7.1Hz, 1H), 1.36 (t, J=7.4Hz, 3H), 1.17 (d, J=7.1Hz, 6H), 1.18 (s, 9H).

### EXAMPLE 9B

A suspension of EXAMPLE 8 (110Kg) and N-chlorosuccinimide (31Kg) in ethyl acetate (785Kg) at 0-5°C was treated with phosphoric acid (2.5Kg) and water (1Kg) while the temperature was kept at less than 5°C, warmed to 22°C, stirred for 3 hours, washed with sodium bicarbonate solution and 10% sodium sulfite solution and concentrated. The concentrate was treated with methyl tert-butyl ether (403Kg), and the slurry was stirred at 35°C for 30 minutes, cooled to 5°C and filtered.

### EXAMPLE 9C

A suspension of EXAMPLE 8 (4.91 g) and N-chlorosuccinimide (1.36 g) in ethyl acetate (500 mL) was treated with trifluoroacetic acid (0.15 mL) over 3 hours then with 5% aqueous sodium bicarbonate (25 mL), separated from the water layer, washed with 10% aqueous sodium hydrogen sulfate (10 mL) and concentrated to 50 mL with a methyl tert-butyl ether (250 mL) azeotrope. The concentrate was dissolved in methyl tert-butyl ether, and the solution was stirred at 45°C until solid formed, cooled to ambient temperature, and filtered. The filtrant was washed with methyl tert-butyl ether and dried under vacuum at 50°C to provide 5.33 g of product as the methyl tert-butyl ether solvate. ¹H NMR (CDCl₃) δ 8.35 (s, 1H), 7.95 (d, J=14.7Hz, 1H), 7.24 (t, J=8.9Hz, 1H), 5.19-5.11 (m, 1H), 4.82-4.72 (m, 2H), 4.39-4.27 (m, 2H), 4.35 (q, J=7.5Hz, 2H), 3.19 (s, 3H), 2.57 (sept, J=7.1Hz, 1H), 1.36 (t, J=7.4Hz, 3H), 1.17 (d, J=7.1Hz, 6H), 1.18 (s, 9H).

### EXAMPLE 10

A solution of N-chlorosuccinimide (25.3Kg) in methyl acetate (419Kg) at 17°C was treated with sulfuric acid (560 g), transferred to a slurry of EXAMPLE 8 (92.7Kg) in ethyl acetate (244Kg) at 17°C while maintaining the reaction temperature at 17°C, quenched/washed with 1.5% aqueous sodium bicarbonate (370Kg), washed with 10% aqueous sodium sulfite (200Kg) and concentrated. The concentrate was dissolved in isopropanol, treated with 4% (w/w) aqueous potassium hydroxide (750Kg), stirred at 50°C until hydrolysis was complete, passed through a polishing filter, treated with 12% aqueous acetic acid (410Kg) and filtered. The filtrant was washed with water and dried at 50°C to provide 73Kg of product. ¹H NMR (CDCl₃) δ 14.63 (brs, 1H), 8.70 (d, J=0.7Hz, 1H), 7.95 (dd, J=9.9, 0.7Hz, 1H), 7.83 (d, J=13.6Hz, 1H), 6.75 (s, 2H), 5.75 (d, J=5.8Hz, 1H), 4.61 (m, 12H), 4.47 (m, 1H), 4.18 (m, 2H).

### Reference EXAMPLE 11A

A solution of EXAMPLE 6 (3.65Kg) and triethyl orthoformate (4.93L) in toluene (18.5L) at reflux was stirred for 1 hour, treated with acetic anhydride (3.50L), stirred for 12-24 hours, cooled to ambient temperature and concentrated with a toluene (8L) azeotrope until no triethyl orthoformate was detected by ¹H NMR (CDCl₃).

### Reference EXAMPLE 11B

A solution of EXAMPLE 4 (2.58Kg) in DMSO (6.75Kg) at 14°C was treated with EXAMPLE 13A in DMSO (9.50Kg) over 1 hour, stirred for 15 minutes, treated with potassium carbonate (2.25Kg), stirred at 60-70°C for 1-2 hours, cooled to 30°C, treated sequentially with acetonitrile (13.3Kg) and 9% aqueous citric acid (20.2Kg), each over 15 minutes, cooled to ambient temperature and filtered. The filtrant was washed with 9% aqueous citric acid (10Kg)/acetonitrile (9.1L) and acetonitrile (2×9.1L) and dried at 40-45°C to provide 4.49Kg of product. ¹H NMR (DMSO-d₆) δ 8.72 (s, 1H), 8.14 (dd, J=11.4, 9.6Hz, 1 H), 8.03 (dd, J=11.2, 9.8Hz, 1H), 7.51 (ddd, J=12.6, 6.6, 1.2Hz, 1H), 6.82 (br s, 2H), 4.23 (q, J=7.5Hz, 2H), 1.28 (t, J=7.5Hz, 3H).

### Reference EXAMPLE 12

A mixture of EXAMPLE 2 (1.46Kg) and potassium bicarbonate (4.66Kg) in N-methylpyrrolidinone (36.8Kg) was stirred at 60°C for 1 hour, treated with EXAMPLE 13B (4Kg), stirred for 3 hours, cooled to ambient temperature, treated with N,N-dimethylaminopyridine (65 g) and acetic anhydride (5.34Kg) while keeping the temperature below 45°C, stirred until the intermediate alcohol was consumed, cooled to ambient temperature, and filtered. The filtrant was washed with N-methylpyrrolidinone (4.3Kg), and the filtrate was warmed to 70°C, treated with water (40.4Kg), cooled to ambient temperature, and filtered. The filtrant was washed with water (2×12Kg) and dried under vacuum at 50°C to provide 4.36 Kg of product. ¹H NMR (DMSO-d₆) δ 8.31 (d, J=0.7 Hz, 1H), 7.81 (dd, J=8.8, 9.9 Hz, 1H), 7.55 (d, J=12.9 Hz, 1H), 6.62 (br s, 2H), 5.75 (dd, J=1.5, 7.3 Hz, 1H), 5.00 (m, 1H), 4.16 (m, 2H), 4.02 (q, J=7.1 Hz, 2H), 3.77 (dd, J=3.3, 9.6 Hz, 2H), 1.87 (s, 3H), 1.07 (t, J=7. Hz, 3H).

### Reference EXAMPLE 13

A mixture of EXAMPLE 12 (1.97Kg) in dichloromethane (15.4Kg) at 0°C was treated with 1,3-dimethyl-5,5-dichlorohydantoin (890 g) in dichloromethane (7.7Kg) over 2.5 hours, stirred for 2 hours, treated with 10% aqueous sodium hydrogen sulfite (10.2Kg), separated from the water layer, washed with water, filtered, and concentrated with an ethyl acetate (4.2Kg) azeotrope. The concentrate was treated with ethyl acetate (9Kg) and heptane (3.5Kg), stirred at 5°C for 2 hours, and filtered. The filtrant was washed with 1:1 ethyl acetate/heptane (1.4Kg) and dried under vacuum to provide 1.84Kg of product. Mp 193-195°C; ¹H NMR (DMSO-d₆, 300 MHz) δ 8.42 (d, J=0.7 Hz, 1H), 7.93 (dd, J=9.9, 1.1 Hz, 1H), 7.75 (d, J=13.9 Hz, 1H), 6.72 (br s, 1H), 5.11 (m, 1H), 4.73 (m, 2H), 4.32 (m, 2H), 4.22 (dd, J=14.0, 7.0 Hz, 2H), 2.07 (s, 3H), 1.26 (t, J=14.0 Hz, 3H).

The preceeding is meant to be illustrative of this invention and not limiting. The scope of this invention is defined in the claims.

## Claims

1. A process for making 1-(6-amino-3,5-difluoropyridin-2-yl)-8-chloro-6-fluoro-7-(3-hydroxyazetidin-1-yl)-4-oxo-1,4-dihydroquinoline-3-carboxylic acid or a salt thereof, comprising:
(a) reacting ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide in ethyl acetate and in the presence of acid and isolating or not isolating the product;
or
reacting ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide in a mixture of methyl acetate and ethyl acetate and in the presence of sulfuric acid and isolating or not isolating the product; and
(b) reacting the product of step (a) and sodium hydroxide.

2. The process of claim 1 further comprising isolating the product of step (b).

3. The process according to claim 1 or 2, wherein ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide are reacted in ethyl acetate.

4. The process according to claim 1 or 2, wherein ethyl 1-(6-amino-3,5-difluoropyridin-2-yl)-6-fluoro-7-(3-(isobutyryloxy)azetidin-1-yl)-4-oxo-1,4-dihydroquinoline-3-carboxylate or a salt thereof and N-chlorosuccinimide are reacted in a mixture of methyl acetate and ethyl acetate.

5. The process according to any of claims 1-3, wherein the acid is sulfuric acid, phosphoric acid, trifluoroacetic acid, or perchloric acid.

6. The process according to claim 5, wherein the acid is sulfuric acid.

7. The process according to claim 5, wherein the acid is phosphoric acid.

8. The process according to claim 5, wherein the acid is trifluoroacetic acid.

9. The process according to claim 5, wherein the acid is perchloric acid.

## Patentansprüche

1. Ein Verfahren zur Herstellung von 1-(6-Amino-3,5-difluorpyridin-2-yl)-8-chlor-6-fluor-7-(3-hydroxyazetidin-1-yl)-4-oxo-1,4-dihydrochinolin-3-carbonsäure oder einem Salz davon, das folgendes umfasst:
(a) Reagieren von Ethyl 1-(6-Amino-3,5-difluorpyridin-2-yl)-6-fluor-7-(3-(isobutyryloxy)azetidin-1-yl)-4-oxo-1,4-dihydrochinolin-3-carboxylat oder einem Salz davon und N-Chlorsuccinimid in Ethylacetat in der Anwesenheit von Säure, und Isolieren oder nicht Isolieren des Produkts;
oder
Reagieren von Ethyl 1-(6-Amino-3,5-difluorpyridin-2-yl)-6-fluor-7-(3-(isobutyryloxy)azetidin-1-yl)-4-oxo-1,4-dihydrochinolin-3-carboxylat oder einem Salz davon und N-Chlorsuccinimid in einer Mischung aus Methylacetat und Ethylacetat und in der Anwesenheit von Schwefelsäure, und Isolieren oder nicht Isolieren des Produkts; und
(b) Reagieren des Produkts aus Schritt (a) und Natriumhydroxid.

2. Das Verfahren gemäß Anspruch 1, weiter umfassend das Isolieren des Produkts aus Schritt (b).

3. Das Verfahren gemäß Anspruch 1 oder 2, worin Ethyl 1-(6-Amino-3,5-difluorpyridin-2-yl)-6-fluor-7-(3-(isobutyryloxy)azetidin-1-yl)-4-oxo-1,4-dihydrochinolin-3-carboxylat oder ein Salz davon und N-Chlorsuccinimid in Ethylacetat reagiert werden.

4. Das Verfahren gemäß Anspruch 1 oder 2, worin Ethyl 1-(6-Amino-3,5-difluorpyridin-2-yl)-6-fluor-7-(3-(isobutyryloxy)azetidin-1-yl)-4-oxo-1,4-dihydrochinolin-3-carboxylat oder ein Salz davon und N-Chlorsuccinimid in einer Mischung aus Methylacetat und Ethylacetat reagiert werden.

5. Das Verfahren gemäß irgendeinem der Ansprüche 1-3, worin die Säure Schwefelsäure, Phosphorsäure, Trifluoressigsäure oder Perchlorsäure ist.

6. Das Verfahren gemäß Anspruch 5, worin die Säure Schwefelsäure ist.

7. Das Verfahren gemäß Anspruch 5, worin die Säure Phosphorsäure ist.

8. Das Verfahren gemäß Anspruch 5, worin die Säure Trifluoressigsäure ist.

9. Das Verfahren gemäß Anspruch 5, worin die Säure Perchlorsäure ist.

## Revendications

1. Procédé de préparation d'acide 1-(6-amino-3,5-difluoropyridin-2-yl)-8-chloro-6-fluoro-7-(3-hydroxyazétidin-1-yl)-4-oxo-1,4-dihydroquinoline-3-carboxylique ou d'un sel de celui-ci, comprenant :
(a) la réaction de 1-(6-amino-3,5-difluoropyridin-2-yl)-6-fluoro-7-(3-(isobutyryloxy)azétidin-1-yl)-4-oxo-1,4-dihydroquinoline-3-carboxylate d'éthyle ou d'un sel de celui-ci et de N-chlorosuccinimide dans de l'acétate d'éthyle et en présence d'acide et l'isolement ou non du produit ;
ou
la réaction de 1-(6-amino-3,5-difluoropyridin-2-yl)-6-fluoro-7-(3-(isobutyryloxy)azétidin-1-yl)-4-oxo-1,4-dihydroquinoline-3-carboxylate d'éthyle ou d'un sel de celui-ci et de N-chlorosuccinimide dans un mélange d'acétate de méthyle et d'acétate d'éthyle et en présence d'acide sulfurique et l'isolement ou non du produit ; et
(b) la réaction du produit de l'étape (a) et d'hydroxyde de sodium.

2. Procédé selon la revendication 1 comprenant en outre l'isolement du produit de l'étape (b).

3. Procédé selon la revendication 1 ou 2, dans lequel le 1-(6-amino-3,5-difluoropyridin-2-yl)-6-fluoro-7-(3-(isobutyryloxy)azétidin-1-yl)-4-oxo-1,4-dihydroquinoline-3-carboxylate d'éthyle ou un sel de celui-ci et du *N-*chlorosuccinimide sont mis à réagir dans de l'acétate d'éthyle.

4. Procédé selon la revendication 1 ou 2, dans lequel du 1-(6-amino-3,5-difluoropyridin-2-yl)-6-fluoro-7-(3-(isobutyryloxy)azétidin-1-yl)-4-oxo-1,4-dihydroquinoline- 3-carboxylate d'éthyle ou un sel de celui-ci et du *N-*chlorosuccinimide sont mis à réagir dans un mélange d'acétate de méthyle et d'acétate d'éthyle.

5. Procédé selon l'une quelconque des revendications 1 à 3, dans lequel l'acide est de l'acide sulfurique, de l'acide phosphorique, de l'acide trifluoroacétique ou de l'acide perchlorique.

6. Procédé selon la revendication 5, dans lequel l'acide est de l'acide sulfurique.

7. Procédé selon la revendication 5, dans lequel l'acide est de l'acide phosphorique.

8. Procédé selon la revendication 5, dans lequel l'acide est de l'acide trifluoroacétique.

9. Procédé selon la revendication 5, dans lequel l'acide est de l'acide perchlorique.
